# EUROPÄISCHE PATENTSCHRIFT

(11) **EP 1 713 755 B1**
(45) Veröffentlichungstag und Bekanntmachung des Hinweises auf die Patenterteilung: **13.01.2010**
(21) Anmeldenummer: 05701122.3
(22) Anmeldetag: 22.01.2005
(51) Int. Cl.: C07C 51/09, C07C 57/32, C07C 67/00, C07D 319/06

(54) **VERFAHREN ZUR HERSTELLUNG VON 2,5-DIMETHYLPHENYLESSIGSÄURE**
METHOD FOR PRODUCING 2,5-DIMETHYLPHENYL ACETIC ACID
PROCEDE POUR PRODUIRE DE L'ACIDE 2,5-DIMETHYLPHENYLACETIQUE

(30) Priorität: 04.02.2004 DE 102004005318
(43) Veröffentlichungstag der Anmeldung: 25.10.2006
(73) Patentinhaber: Bayer CropScience AG, 40789 Monheim (DE)
(72) Erfinder: HIMMLER, Thomas, 51519 Odenthal (DE)
(86) Internationale Anmeldenummer: PCT/EP2005/000617
(87) Internationale Veröffentlichungsnummer: WO 2005/075401

(56) Entgegenhaltungen:
- EP-A- 0 034 871
- EP-A- 0 101 124
- DE-A1- 3 322 459
- C. GIORDANO ET AL.: "Synthesis of Anti-inflammatory .alpha.-Arylalkanoic acid by 1,2-Aryl Shift" ANGEW. CHEM. INT. ED. ENGL., Bd. 23, 1984, Seiten 413-419, XP002332663 in der Anmeldung erwähnt
- A. KUMAR ET AL.: "FACLIE 1,2-ARYL MIGRATION OF 2-HALOMETHYL-2-(4'-HYDROXYPHENYL) KETALS: A NOVEL SINGLE STEP SYNTHESIS OF 4-HYDROXYPHENYLACETIC ACID & ITS DERIVATIVES" SYNTHETIC COMMUNICATIONS, Bd. 27, Nr. 7, 1997, Seiten 1133-1141, XP009047841 in der Anmeldung erwähnt

## Beschreibung

Die vorliegende Erfindung betrifft ein Verfahren zur Herstellung von 2,5-Dimethylphenylessigsäure.

2,5-Dimethylphenylessigsäure ist eine bekannte Verbindung (beispielsweise aus: Z.J. Vejdelek et al., Collect. Czech. Chem. Commun. 29 (1964) 776-94). Die Herstellung kann beispielsweise ausgehend von 2,5-Dimethylphenylacetophenon durch eine Willgerodt-Kindler-Reaktion erfolgen (H.E. Zaugg et al., J. Amer. Chem. Soc. 70 (1948) 3224-8). Bei dieser Methode fallen jedoch große Mengen schwefelhaltiger Abfälle an. Außerdem können stark geruchsbelästigende leichtflüchtige Schwefelverbindungen auftreten.

Eine weitere Methode zur Herstellung von 2,5-Dimethylphenylessigsäure geht von 2,5-Dimethylbenzylbromid aus. Man stellt daraus z.B. mit Natriumcyanid das entsprechende Nitril her, welches anschließend verseift wird. Das benötigte 2,5-Dimethylbenzylbromid kann beispielsweise durch Brommethylierung von p-Xylol mit Formaldehyd und Bromwasserstoff hergestellt werden (H. Hart et al, Tetrahedron Letters 1975, 4639-42; J.M. Khurana und G.C. Maikap, J. Ind. Chem. Soc. 76 (1999) 216-7). Hierbei ist allerdings nachteilig, dass unerwünschte Nebenreaktionen zu mehrfach brommethylierten Produkten stattfinden können. Außerdem kann das Auftreten von Bisbrommethyl-ether nicht ausgeschlossen werden, so dass hier technisch aufwendige Sicherheitsmaßnahmen ergriffen werden müssen.

Eine weitere bekannt gewordene Möglichkeit zur Herstellung von 2,5-Dimethylphenylessigsäure besteht darin, von 2,5-Dimethylbenzylchlorid auszugehen, daraus das Nitril herzustellen (J. Amer. Chem. Soc. 58 (1936) 629-35; J. Org. Chem. 33 (1968) 2338-42) und dieses dann zu verseifen. 2,5-Dimethylbenzylchlorid ist ebenfalls bekannt und kann durch Chlormethylierung von p-Xylol hergestellt werden (Z.J. Vejdelek et al., Collect. Czech. Chem. Commun. 29 (1964) 776-94). Allerdings ist die Chlormethylierung wegen der Möglichkeit, dass dabei der hochtoxische Bis-chlormethyl-ether auftritt, eine nur mit hohem technischen Aufwand und Kosten durchführbare Methode. Außerdem liefert diese Methode die 2,5-Dimethylphenylessigsäure nur in unbefriedigenden Ausbeuten (z.B. 38% der Theorie über 3 Stufen. nach Z.J. Vejdelek et al., Collect. Czech. Chem. Commun. 29 (1964) 776-94).

Alle bisher bekannt gewordenen Methoden zur Herstellung von 2,5-Dimethylphenylessigsäure weisen demnach z.T. erhebliche Mängel und Nachteile auf, die die Herstellung von 2,5-Dimethylphenylessigsäure erschweren. Da allgemein Phenylessigsäuren, und unter ihnen speziell auch die 2,5-Dimethylphenylessigsäure, wichtige Vorprodukte beispielsweise für Wirkstoffe im Pflanzenschutz sind (vgl. WO 97/36868), besteht Bedarf an einer technisch einfachen und hocheffizienten Methode zur Herstellung von 2,5-Dimethylphenylessigsäure.

Es wurde nun gefunden, dass man 2,5-Dimethylphenylessigsäure überraschenderweise in sehr hoher Ausbeute und Reinheit erhält, indem man zunächst p-Xylol mit Chloracetylchlorid in einer Friedel-Crafts-Reaktion zu 2-Chlor-1-(2,5-dimethylphenyl)-ethanon der Formel (I) umsetzt, aus diesem Keton mit einem Diol der allgemeinen Formel (II) das entsprechende Ketal der allgemeinen Formel (III) herstellt, dieses dann zu einem Gemisch der entsprechenden 2,5-Dimethylphenylessigsäure-hydroxyalkylester der allgemeinen Formel (IV) und Bis-(2,5-dimethylphenylessigsäure)-diester der allgemeinen Formel (V) umlagert und diese schließlich zur 2,5-Dimethylphenylessigsäure verseift.

Das erfindungsgemäße Verfahren kann durch folgendes Schema veranschaulicht werden:

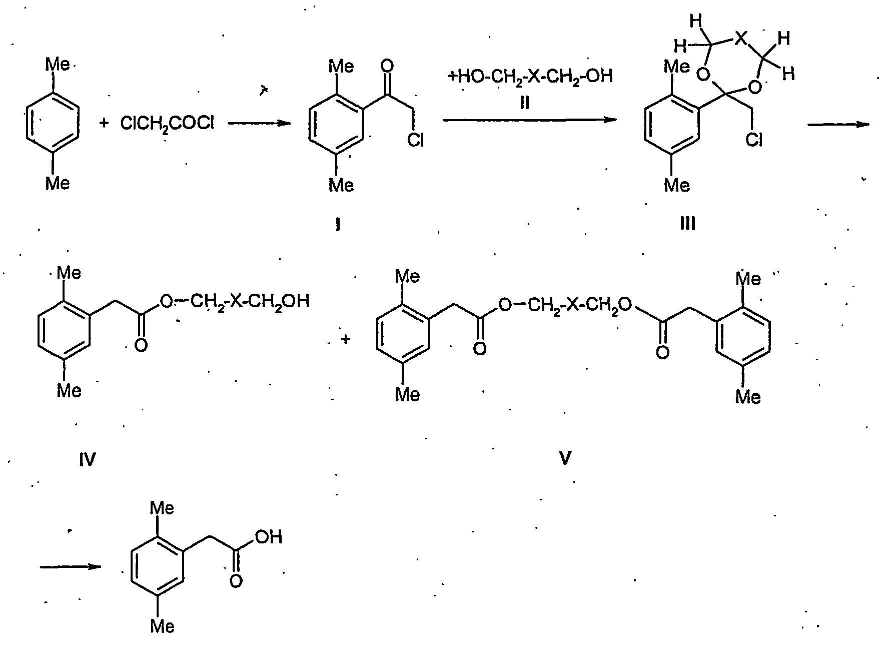

Die Verbindung der Formel (I) ist bekannt (siehe beispielsweise F. Kunckell, Chem. Ber. 30 (1897) 577-579) und kann außer durch Friedel-Crafts-Acylierung beispielsweise auch durch Chlorierung von 2,5-Dimethylacetophenon hergestellt werden.

In den allgemeinen Formeln (II), (III), (IV) und (V) steht
- X: für eine direkte Einfachbindung, CH₂, CHCH₃, CHC₂H₅, C(CH₃)₂ oder C(C₂H₅)₂.

Die Verbindungen der Formel (II) sind bekannt und kommerziell erhältlich.

Ebenfalls Gegenstand der vorliegenden Erfindung sind die neuen Verbindungen der allgemeinen Formel (III), in der
- X: für eine direkte Einfachbindung, CH₂, CHCH₃, CHC₂H₅, C(CH₃)₂ oder C(C₂H₅)₂ steht.

Bevorzugt sind Verbindungen der allgemeinen Formel (III), bei denen
- X: für eine direkte Einfachbindung, CH₂, C(CH₃)₂ oder C(C₂H₅)₂ steht.

Besonders bevorzugt sind Verbindungen der allgemeinen Formel (III), bei denen
- X: für eine direkte Einfachbindung, C(CH₃)₂ oder C(C₂H₅)₂ steht.

Überraschenderweise kann nach dem erfindungsgemäßen Verfahren die 2,5-Dimethylphenylessigsäure auf einfachere Weise, in besserer Selektivität und in höherer Ausbeute als nach den früher bekannt gewordenen Verfahren hergestellt werden.

Als Friedel-Crafts-Katalysatoren zur Herstellung der Verbindung der Formel (I) nach dem erfindungsgemäßen Verfahren können beispielsweise Aluminiumchlorid, Eisen(III)-chlorid, Zinntetrachlorid oder Zeolithe eingesetzt werden.

Bevorzugt wird als Friedel-Crafts-Katalysator Aluminiumchlorid eingesetzt.

Die Menge an nach dem erfindungsgemäßen Verfahren einzusetzendem Friedel-Crafts-Katalysator ist nicht kritisch. So können beispielsweise 0,8 bis 1,2 Mol Katalysator pro Mol Chloracetylchlorid eingesetzt werden. Bevorzugt sind 0,9 bis 1,1 Mol Katalysator pro Mol Chloracetylchlorid.

Als Lösungsmittel für die Friedel-Crafts-Reaktion werden nach dem erfindungsgemäßen Verfahren weitgehend inerte Lösungsmittel wie beispielsweise Nitrobenzol, Schwefelkohlenstoff, Methylenchlorid, 1,2-Dichlorethan oder p-Xylol selbst eingesetzt. Bevorzugt sind Schwefelkohlenstoff, 1,2-Dichlorethan und p-Xylol. Besonders bevorzugt ist p-Xylol.

Die Menge an nach dem erfindungsgemäßen Verfahren einzusetzendem Chloracetylchlorid ist nicht kritisch und kann in weiten Grenzen variiert werden. Bei Verwendung eines Lösungsmittels kann man beispielsweise 0,8 bis 1,2 Mol Chloracetylchlorid pro Mol p-Xylol einsetzen. Bevorzugt sind 0,9 bis 1,1 Mol Chloracetylchlorid pro Mol p-Xylol.

Verwendet man einen Überschuss an p-Xylol als Lösungsmittel, wird das Verhältnis von Chloracetylchlorid zu p-Xylol naturgemäß deutlich kleiner sein.

Da bekannt ist (siehe beispielsweise L. Friedman und R. Koca, J. Org. Chem. 33 (1968) 1255-7), dass p-Xylol durch einen Friedel-Crafts-Katalysator wie AlCl₃ isomerisiert werden kann, geht man zweckmäßigerweise so vor, dass p-Xylol und Chloracetylchlorid vorgelegt werden und man den Friedel-Crafts-Katalysator zudosiert.

Der erste Schritt des erfindungsgemäßen Verfahrens kann bei Temperaturen zwischen -20 und +60°C durchgeführt werden. Bevorzugt sind Temperaturen zwischen -10 und +30°C.

Die Reaktionszeiten des ersten Schritts des erfindungsgemäßen Verfahrens betragen zwischen 1 und 24 Stunden.

Im zweiten Schritt des erfindungsgemäßen Verfahrens wird das Ketal der allgemeinen Formel (III) durch Erhitzen des Ketons der Formel (I) mit einem Diol der allgemeinen Formel (II) in Gegenwart eines Katalysators hergestellt.

Als Diole der allgemeinen Formel (II) seien beispielhaft Ethylenglykol, 1,2-Propandiol (Propylenglykol), 1,3-Propandiol (Trimethylenglykol), 2,2-Dimethyl-1,3 propandiol (Neopentylglykol) und 2,2-Diethyl-1,3-propandiol genannt. Bevorzugt sind Ethylenglykol und Neopentylglykol. Besonders bevorzugt ist Neopentylglykol.

Als Katalysatoren für den zweiten Schritt des erfindungsgemäßen Verfahrens kommen Säuren wie Salzsäure, Schwefelsäure, Phosphorsäure, Methylsulfonsäure, p-Toluolsulfonsäure, saure Ionenaustauscher u.a. in Betracht. Bevorzugt sind Salzsäure, Schwefelsäure und p-Toluolsulfonsäure.

Als Lösungsmittel für den zweiten Schritt des erfindungsgemäßen Verfahrens sind inerte organische Lösungsmittel wie beispielsweise aliphatische und aromatische Kohlenwasserstoffe, sowie die Diole selbst geeignet. Bevorzugt werden aromatische Kohlenwasserstoffe verwendet, besonders bevorzugt Xylol selbst.

Bei der Herstellung des Ketals der allgemeinen Formel (III) in Gegenwart eines sauren Katalysators wird zweckmäßigerweise so vorgegangen, dass das Reaktionswasser mit dem Lösungsmittel, bevorzugt dem aromatischen Kohlenwasserstoff, azeotrop abdestilliert wird.

Die Umlagerung (1,2-Arylverschiebung) des Ketals der allgemeinen Formel (III) im dritten Schritt des erfindungsgemäßen Verfahrens zu den 2,5-Dimethylphenylessigsäureestern der allgemeinen Formeln (IV) und (V) erfolgt in prinzipiell bekannter Weise durch Erhitzen in einem polarprotischem Lösungsmittel in Gegenwart einer schwachen Base (C. Giordano et al., Angew. Chem. 96 (1984) 413-9; EP-A 101 124). Eine weitere Methode zur 1,2-Arylverschiebung des Ketals der allgemeinen Formel (III) besteht im Erhitzen in Gegenwart einer Lewis-Säure wie beispielsweise FeCl₂, FeCl₃, CaCl₂, CuCl₂ oder ZnCl₂ (EP-A 034 871). Weiterhin ist bekannt geworden, dass die 1,2-Arylverschiebung in substituierten 1,3-Dioxanketalderivaten mit katalytischen Mengen eines im Reaktionsgemisch löslichen Zinkcarboxylatsalzes wie beispielsweise Zink-2-ethylhexanoat durchgeführt werden kann (DE-A 33 22 459). Die letztgenannten Verfahren haben jedoch den Nachteil, dass die Katalysatoren wie z.B. Zinkverbindungen nach aufwendigen Methoden (Ausfällen; Behandeln des Reaktionsgemisches mit Aktivkohle) entfernt werden müssen. Außerdem entstehen chlorierte Alkohole als Abfallprodukte, die entsorgt werden müssen.

Weiterhin ist bereits bekannt geworden, dass sich 2-Halomethyl-2-(4`-hydroxyphenyl)-ketale durch Erhitzen mit Natronlauge zu 4-Hydroxyphenylessigsäure-Derivaten umlagern lassen (A. Kumar und R. A. Rane, Synthetic Commun. 27 (1997) 1133-41). Diese Reaktion gelingt jedoch mit den chlorsubstituierten Ketalen schlechter als mit den bromsubstituierten Ketalen. Außerdem liegt durch die 4-Hydroxy-Substitution am Aromaten eine besondere Situation vor, da das im alkalischen Milieu vorliegende Phenolation infolge chinoider Grenzstrukturen die Reaktivität an der Ketalgruppe stark beeinflussen kann. Eine derartige Beeinflussung ist beispielsweise auch bei der Reduktion von 4-Hydroxy-mandelsäuren bekannt (J.C. Vallejos et al., Bull. Soc. Chim. Fr. 134 (1997) 101-4). Es war daher keineswegs von vornherein zu erwarten, dass diese Verfahrensvariante auch mit anderen als 4-Hydroxy-phenyl-substituierten Chlormethylketalen gelingen würde.

Als polar-protische Lösungsmittel für den dritten Schritt des erfindungsgemäßen Verfahrens können Wasser, Alkohole, Diole, Polyole und Mischungen derselben benutzt werden.

In dem Gemisch der Verbindungen der Formeln (IV) und (V) kann in Abhängigkeit vom verwendeten Lösungsmittel im dritten Schritt des erfindungsgemäßen Verfahrens aufgrund von Umesterungen der Rest X innerhalb der oben angegebenen Definition auch eine andere Bedeutung als in den Formeln (II) bzw. (III) haben. Führt man beispielsweise das Verfahren im zweiten Schritt unter Verwendung von Neopentylglykol (X = C(CH₃)₂ in Verbindung der Formel (II) und (III)) durch, und verwendet man im dritten Schritt des erfindungsgemäßen Verfahrens dann Ethylenglykol als Lösungsmittel, so kann das Gemisch der Verbindungen der Formeln (IV) und (V) sowohl solche mit X = C(CH₃)₂ als auch solche mit X = direkte Einfachbindung enthalten.

Die Menge an Lösungsmittel kann in weiten Grenzen variiert werden. Typischerweise werden zwischen 200 ml und 2000 ml Lösungsmittel pro Mol Ketal eingesetzt.

Als Basen können im dritten Schritt des erfindungsgemäßen Verfahrens beispielsweise Alkalisalze der Ameisensäure, Essigsäure, Propionsäure oder Benzoesäure, Alkaliphosphate, -carbonate und -hydrogencarbonate verwendet werden.

Die Basen können in Mengen von 1 bis 3 Mol pro Mol Ketal eingesetzt werden. Bevorzugt sind 1,2 bis 1,6 Mol pro Mol Ketal.

Es ist nach dem erfindungsgemäßen Verfahren auch möglich, nur katalytische Mengen eines Alkalisalzes der Ameisensäure, Essigsäure, Propionsäure oder Benzoesäure einzusetzen und dafür zusätzlich Natrium- oder Kaliumhydroxid zuzusetzen. Bevorzugt werden 0,1 bis 0,3 Mol pro Mol Ketal in Kombination mit 1 bis 3 Mol Natrium- oder Kaliumhydroxid pro Mol Ketal verwendet.

Besonders bevorzugt werden 0,1 bis 0,3 Mol Natriumacetat pro Mol Ketal in Kombination mit 1 bis 3 Mol Natriumhydroxid pro Mol Ketal verwendet.

Außerdem ist es nach dem erfindungsgemäßen Verfahren auch möglich, auf den Einsatz eines Alkalisalzes der Ameisensäure, Essigsäure, Propionsäure oder Benzoesäure völlig zu verzichten und die Reaktion des dritten Schrittes allein durch Zugabe eines Alkalihydroxid zu bewirken. Dabei kann das Alkalihydroxid in fester Form oder als wässrige Lösung eingesetzt werden.

Bevorzugt werden Kalium- und Natrimhydroxid verwendet.

Bei diesen beiden Ausführungsformen des dritten Schrittes des erfindungsgemäßen Verfahrens erfolgt direkt auch die Verseifung der Zwischenprodukte der allgemeinen Formeln (IV) und (V), so dass der sonst übliche 4. Schritt des Verfahrens entfallen kann.

Der dritte Schritt des erfindungsgemäßen Verfahrens zur Herstellung von 2,5-Dimethylphenylessigsäure kann bei Temperaturen zwischen 100 und 250°C durchgeführt werden. Bevorzugt sind Temperaturen zwischen 150 und 230°C, besonders bevorzugt zwischen 170 und 220°C.

Die Reaktionszeiten des dritten Schritts des erfindungsgemäßen Verfahrens betragen zwischen 1 und 24 Stunden.

Im vierten Schritt des erfindungsgemäßen Verfahrens werden die Ester der allgemeinen Formeln (IV) und (V) nach bekannten Methoden der organischen Chemie zur 2,5-Dimethylphenylessigsäure verseift.

Bevorzugt erfolgt die Verseifung durch Erhitzen mit Natronlauge.

Das erfindungsgemäße Verfahren zur Herstellung von 2,5-Dimethylphenylessigsäure wird bevorzugt so gestaltet, dass alle Schritte nacheinander ohne Isolierung der Zwischenprodukte durchgeführt werden. Dadurch entfallen zeit- und kostenintensive Aufarbeitungs- und Reinigungsschritte wie Kristallisieren, Filtrieren, Trocknen usw.

Es kann als ausgesprochen überraschend bezeichnet werden, dass trotz fehlender Reinigungsoperationen auf den Zwischenstufen die 2,5-Dimethylphenylessigsäure nach dem erfindungsgemäßen Verfahren nicht nur in sehr hoher Ausbeute sondern auch hervorragender Reinheit erhalten wird.

Dementsprechend wird das erfindungsgemäße Verfahren zur Herstellung von 2,5-Dimethylphenylessigsäure bevorzugt folgendermaßen durchgeführt:
Im ersten Schritt des erfindungsgemäßen Verfahrens wird p-Xylol mit Chloracetylchlorid in Gegenwart von Aluminiumchlorid nach Friedel-Crafts zum 2-Chlor-1-(2,5-dimethylphenyl)-ethanon umgesetzt. Als Lösungsmittel dient dabei ein Überschuss an p-Xylol. Nach Aufarbeitung mit Wasser und Salzsäure nach bekannten Methoden der organischen Chemie wird die organische Phase (= Lösung von 2-Chlor-1-(2,5-dimethylphenyl)-ethanon in Xylol) in die nächste Stufe eingesetzt.

Im zweiten Schritt des erfindungsgemäßen Verfahrens wird die Lösung von 2-Chlor-1-(2,5-dimethylphenyl)-ethanon in Xylol mit Neopentylglykol und einer katalytischen Menge p-Toluolsulfonsäure versetzt. Anschließend wird am Wasserabscheider zum Rückfluss erhitzt, bis mindestens die erwartete theoretische Menge Wasser abgetrennt wurde. Ohne weitere Aufarbeitung kann das Reaktionsgemisch in die nächste Stufe eingesetzt werden.

In einer Ausführungsform des dritten Schrittes des erfindungsgemäßen Verfahrens wird die Lösung von 2-Chlormethyl-5,5-dimethyl-2-(2,5-dimethylphenyl)-[1,3]dioxan in Xylol zunächst mit Natriumacetat versetzt. Anschließend wird Ethylenglykol hinzugegeben und dann das Xylol abdestilliert, bis eine Reaktionstemperatur von etwa 180 bis 190°C erreicht ist. Diese Temperatur wird für 4 bis 7 Stunden gehalten. Anschließend lässt man auf etwa 90°C abkühlen und setzt das Reaktionsgemisch ohne weitere Aufarbeitung in die nächste Stufe ein.

In einer Ausführungsform des vierten Schrittes des erfindungsgemäßen Verfahrens zur Herstellung von 2,5-Dimethylphenylessigsäure wird das Reaktionsgemisch aus der dritten Stufe bei etwa 90 bis 95°C mit Natronlauge versetzt und 1 bis 2 Stunden auf 100 bis 105°C erhitzt. Anschließend lässt man auf Raumtemperatur abkühlen, versetzt das Reaktionsgemisch mit Wasser, stellt durch Zugabe einer Säure wie beispielsweise Salz- oder Schwefelsäure sauer und isoliert dann die 2,5-Dimethylphenylessigsäure durch Filtration. Durch Waschen mit Wasser und anschließendes Trocknen nach üblichen Methoden wird die 2,5-Dimethylphenylessigsäure in hoher Ausbeute und Reinheit erhalten.

Die Herstellung von 2,5-Dimethylphenylessigsäure nach dem erfindungsgemäßen Verfahren soll durch die folgenden Herstellungsbeispiele erläutert werden:

### Herstellungsbeispiele für die einzelnen Stufen

### Beispiel 1: 2-Chlor-1-(2,5-dimethylphenyl)-ethanon

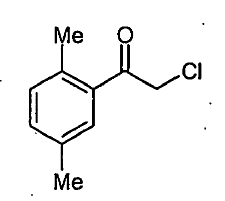

Zu einer Mischung aus 800 g p-Xylol und 226 g [2 Mol] Chloracetylchlorid werden bei 12-15°C innerhalb von ca. 75 Minuten 293,2 g [2,2 Mol] Aluminiumchlorid dosiert. Man rührt noch 2 Stunden bei 12-15°C, lässt auf Raumtemperatur kommen, rührt noch 30 Minuten bei Raumtemperatur und gießt dann das Reaktionsgemisch in 3000 ml Eiswasser mit 70 g konz. Salzsäure. Die trübe organische Phase wird abgetrennt, die wässrige Phase dreimal mit je 300 ml Essigester, die vereinigten organischen Phasen zweimal mit je 200 ml Wasser und einmal mit 100 ml gesättigter wässriger NaCl-Lösung ausgeschüttelt. Die organische Phase wird getrocknet, einrotiert und bis 70°C Badtemperatur / 1 mbar andestilliert. Es resultieren 363,6 g gelbliches Öl, das nach GC 97,2% Zielprodukt enthält (96,8% der Theorie).
¹H-NMR (400 MHz, CDCl₃): δ = 2,37 (s, 3H), 2,47 (s, 3H), 4,63 (s, 2H), 7,06 (d, 7,8Hz, 1H), 7,23-7,26 (m, 1H), 7,4 (s, 1H)ppm.

### Beispiel 2: 2-Chlormethyl-5,5-dimethyl-2-(2,5-dimethylphenyl)-[1,3]dioxan

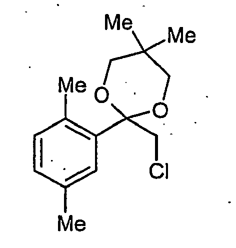

0,5 Mol 2-Chlor-1-(2,5-dimethylphenyl)-ethanon, 104 g [1 Mol] 2,2-Dimethyl-1,3-propandiol (Neopentylglykol) und 9,5 g [0,05 Mol] pTsOH-Hydrat werden in 500 ml Xylol am Wasserabscheider bis zur Beendigung der Wasserbildung gekocht (ca. 4 Stunden). Das Reaktionsgemisch wird bei Raumtemperatur mit 200 ml Wasser und 30 ml Xylol versetzt. Durch Zugabe von 50 ml gesättigter wässriger NaCl-Lösung wird eine bessere Phasentrennung erreicht. Die organische Phase wird dann nochmals mit 100 ml Wasser und 100 ml gesättiger wässriger NaCl-Lösung ausgeschüttelt, getrocknet und eingeengt. Man erhält ein Öl, das nach Ausgießen auf ein Blech kristallisiert. Ausbeute: 136,5 g mit lt. GC 93,1% Zielprodukt (94,6% der Theorie).
¹H-NMR (400 MHz, CDCl₃): δ = 0,62 (s, 3H), 1,37 (s, 3H), 2,34 (s, 3H), 2,37 (s, 3H), 3,43-3,52 (m, 4H), 3,59 (s, 2H), 7,06-7,10 (m, 2H), 7,30 (s, 1H)ppm.
Schmelzpunkt: 80,5-81,5°C

### Beispiel 3: 2,5-Dimethylphenylessigsäure

Eine Mischung aus. 4,1 g [0,05 Mol] Natriumacetat und 10,75 g [0,04 Mol] 2-Chlormethyl-5,5-dimethyl-2-(2,5-dimethylphenyl)-[1,3]dioxan in 50 ml Ethylenglykol wird 5 Stunden auf 180 bis 185°C erhitzt. Anschließend lässt man auf 90 bis 95°C abkühlen, gibt 20 ml 30%ige Natronlauge hinzu und erhitzt 1 Stunde auf 100 bis 105°C. Bei Raumtemperatur wird das Reaktionsgemisch mit 80 ml Wasser verdünnt und zweimal mit je 10 ml Methylenchlorid ausgeschüttelt. Die wässrige Phase wird dann mit konz. Salzsäure auf pH 1 gestellt, der Feststoff abgesaugt, zweimal mit je 20 ml Wasser gewaschen und getrocknet. Man erhält 6,20 g weißen Feststoff mit einer Reinheit von 99,3% (GC). Damit ergibt sich eine Ausbeute von 93,7% der Theorie.

### Bespiel 4: 2-Chlormethyl-2-(2,5-dimethylphenyl)-[1,3]dioxolan

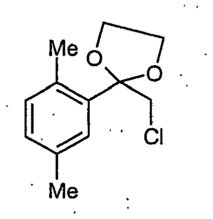

Eine Mischung aus 18,3 g [0,1 Mol] 2-Chlor-1-(2,5-dimethylphenyl)-ethanon, 12,6 g [0,2 Mol] Methylenglykol, 1,9 g [0,01 Mol] pTsOH-Hydrat und 100 ml Xylol wird ca. 3 Stunden am Wasserabscheider zum Sieden erhitzt. Das Reaktionsgemisch wird anschließend bei Raumtemperatur mit 40 ml Wasser und 20 ml gesättigter wässriger NaCl-Lösung versetzt. Nach Zugabe von 20 ml Xylol wird die organische Phase abgetrennt und mit je 20 ml Wasser und gesättigter wässriger NaCl-Lösung ausgeschüttelt. Man trocknet die organische Phase über Natriumsulfat und engt im Vakuum ein. Es resultieren 18,35 g Öl mit lt. GC 95,3% Produkt (77,2% der Theorie.).
GC/MS: m/e = 226 (M⁺ mit ³⁵Cl, < 1%), 177 (M - CH₂Cl, 100%), 133 (177 - OCH₂CH₂, 50%), 105 (133 - CO, 20%).

### Beispiel 5: 2-Chlormethyl-2-(2,5-dimethylphenyl)-[1,3]dioxan

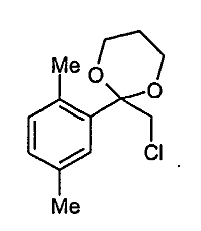

Eine Mischung aus 18,3 g [0,1 Mol] 2-Chlor-1-(2,5-dimethylphenyl)-ethanon, 15,2 g [0,2 Mol] Trimethylenglykol, 1,9 g [0,01 Mol] pTsOH-Hydrat und 150 ml Xylol wird ca. 5 Stunden am Wasserabscheider zum Sieden erhitzt. Anschließend wird das Xylol teilweise abdestilliert und das Reaktionsgemisch bei Raumtemperatur mit 30 ml gesättigter wässriger NaCl-Lösung und zweimal je 100 ml Wasser ausgeschüttelt. Man trocknet die organische Phase über Natriumsulfat, engt im Vakuum ein und destilliert bis ca. 87°C bei 0,25 mbar alle flüchtigen Anteile ab. Es bleiben 22,9 g Rückstand mit nach GC 83,9% Produkt (79,8% der Theorie).
GC/MS: m/e = 240 (M⁺ mit ³⁵Cl, < 1%), 191 (M - CH₂Cl, 100%), 133 (177 - OCH₂CH₂CH₂, 100%), 105 (133 - CO, 25%).

### Beispiel 6: 2-Chlormethyl-4-methyl-2-(2,5-dimethylphenyl)-[1,3]dioxolan

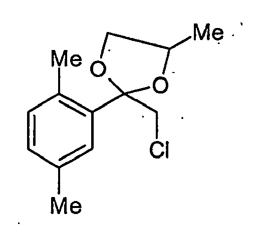

Eine Mischung aus 36,5 g [0,2 Mol] 2-Chlor-1-(2,5-dimethylphenyl)-ethanon, 22,8 g [0,3 Mol] Propylenglykol, 3,6 g [0,02 Mol] pTsOH-Hydrat und 150 ml Xylol wird ca. 1,5 Stunden am Wasserabscheider zum Sieden erhitzt. Anschließend wird das Xylol teilweise abdestilliert und das Reaktionsgemisch bei Raumtemperatur mit zweimal je 100 ml Wasser ausgeschüttelt. Man trocknet die organische Phase über Natriumsulfat, engt im Vakuum ein und destilliert bis ca. 50°C / 0,2 mbar flüchtige Anteile ab. Es verbleiben 39,65 g eines orangefarbenen Öls mit nach GC 93,0% Produkt (76,7% der Theorie)
GC/MS: m/e = 240 (M⁺ mit ³⁵Cl, < 1%), 191 (M - CH₂Cl, 90%), 133 (177 - OCH₂CHMe-, 100%), 105 (133 - CO, 25%).

### Beispiel 7: Durchführung des erfindungsgemäßen Verfahrens mit Kaliumacetat in 4 Schritten

### 1. Schritt

Bei 20-25°C werden zu einer Mischung aus 400 g p-Xylol und 113 g [1 Mol] Chloracetylchlorid innerhalb von 70 Minuten 146,6 g [1,1 Mol] AlCl₃ eingetragen. Man rührt für 2 Stunden bei 20-25°C und versetzt des Reaktionsgemisch dann mit 750 ml eiskaltem Wasser und 35 g konz. Salzsäure. Nach 30 Minuten Rühren wird die organische Phase abgetrennt und in den 2. Schritt eingesetzt.

### 2. Schritt

Die organische Phase aus dem 1. Schritt (469,2 g) wird mit 135,5 g [1,3 Mol] Neopentylglykol und 19 g [0,1 Mol] p-Toluolsulfonsäurehydrat versetzt. Das Gemisch wird am Wasserabscheider für ca. 6 Stunden zum Rückfluss erhitzt, wobei ca. 34 ml wässrige Phase abgetrennt werden. Zusätzlich werden noch 340 ml Xylol abdestilliert. Das Gemisch wird dann ohne weitere Aufarbeitung in den 3. Schritt eingesetzt.

### 3. Schritt

Das noch warme und flüssige Reaktionsgemisch aus dem 2. Schritt wird mit 137,4 g [1,4 Mol] Kaliumacetat und 1250 ml Ethylenglykol versetzt. Anschließend wird für 5 Stunden auf 183 bis 189°C erhitzt, wobei kleine Mengen Destillat abgenommen werden. Das Gemisch wird dann ohne weitere Aufarbeitung in den 4. Schritt eingesetzt.

### 4. Schritt

Man lässt das Reaktionsgemisch aus dem 3. Schritt auf 90 bis 95°C abkühlen, versetzt mit 500 ml 30%iger Natronlauge und erwärmt für 1 Stunde auf 100 bis 105°C. Anschließend verdünnt man das Reaktionsgemisch mit 2000 ml Wasser und extrahiert zweimal mit je 150 ml Methylenchlorid. Die wässrige Phase wird dann mit konz. Salzsäure auf pH 1 gestellt, der ausgefallenen Feststoff abgesaugt, zweimal mit je 500 ml Wasser gewaschen und getrocknet. Man erhält 117 g Feststoff mit einer Reinheit nach GC von 95,2%. Damit ergibt sich eine Ausbeute von 67,5% d.Th. über 4 Schritte, d.h. im Durchschnitt ca. 90 bis 91% je Stufe.

### Beispiel 8: Durchführung des erfindungsgemäßen Verfahrens mit katalytischer Menge Natriumacetat und Natronlauge in 3 Schritten

### 1. Schritt

Bei 20-25°C werden zu einer Mischung aus 400 g p-Xylol und 113 g [1 Mol] Chloracetylchlorid innerhalb von 70 Minuten 146,6 g [1,1 Mol] AlCl₃ eingetragen. Man rührt für 2 Stunden bei 20-25°C und versetzt des Reaktionsgemisch dann mit 750 ml eiskaltem Wasser und 35 g konz. Salzsäure. Nach 30 Minuten Rühren wird die organische Phase abgetrennt und in den 2. Schritt eingesetzt.

### 2. Schritt

Die organische Phase aus dem 1. Schritt (473,2 g) wird mit 135,5 g [1,3 Mol] Neopentylglykol und 19 g [0,1 Mol] p-Toluolsulfonsäurehydrat versetzt. Das Gemisch wird am Wasserabscheider für ca. 6 Stunden zum Rückfluss erhitzt, wobei ca. 29 ml wässrige Phase abgetrennt werden. Das Gemisch wird dann ohne weitere Aufarbeitung in den 3. Schritt eingesetzt.

### 3. Schritt

Das Gemisch aus dem 2. Schritt wird mit 16,4 g [0,2 Mol] Natriumacetat versetzt. Nach 30 Minuten Rühren bei Raumtemperatur werden 1250 ml Ethylenglykol hinzugegeben. Das Gemisch wird bei 150 bis 170 mbar zum Sieden erhitzt und man destilliert 295 g ab. Anschließend werden wieder 30 ml Ethylenglykol und dann 267 g 30%ige Natronlauge [entspricht 3 Mol NaOH] hinzugegeben. Man erhitzt das Reaktionsgemisch in einem Autoklaven unter Eigendruck für 6 Stunden auf 190 bis 195°C. Nach Abkühlen auf Raumtemperatur wird der Autoklav entleert und das Reaktionsgemisch mit 2500 ml Wasser verdünnt. Es wird dreimal mit je 300 ml Methyltertiärbutyl-ether (MTBE) ausgeschüttelt und die wässrige Phase dann mit konz. Salzsäure auf pH 1 gestellt. Der ausgefallene Feststoff wird abgesaugt, zweimal mit je 1000 ml Wasser gewaschen und getrocknet. Man erhält 124,4 g weißen Feststoff mit einer Reinheit von 96,9%. Das ist eine Ausbeute von 73,4% der Theorie über 3 Schritte, d.h. im Durchschnitt ca. 90% der Theorie je Schrift.

### Bespiel 9: Durchführung des erfindungsgemäßen Verfahrens mit Natronlauge in 3 Schritten

### 1. Schritt

Bei 20-25°C werden zu einer Mischung aus 400 g p-Xylol und 113 g [1 Mol] Chloracetylchlorid innerhalb von 60 Minuten 146,6 g [1,1 Mol] AlCl₃ eingetragen. Man rührt für 2 Stunden bei 20-25°C und versetzt des Reaktionsgemisch dann mit 750 ml eiskaltem Wasser und 35 g konz. Salzsäure. Nach 30 Minuten Rühren wird die organische Phase abgetrennt und in den 2. Schritt eingesetzt.

### 2. Schritt

Die organische Phase aus dem 1. Schritt (476,7 g) wird mit 135,5 g [1,3 Mol] Neopentylglykol und 19 g [0,1 Mol] p-Toluolsulfonsäurehydrat versetzt. Das Gemisch wird am Wasserabscheider für ca. 7 Stunden zum Rückfluss erhitzt, wobei ca. 37 ml wässrige Phase abgetrennt werden. Anschließend werden unter Vakuum 315,8 g Destillat abgenommen. Der Rückstand wird dann in den 3. Schritt eingesetzt.

### 3. Schritt

Der aus dem 2. Schritt erhaltene Rückstand (315,6 g) wird mit 1000 ml Ethylenglykol und 267 g 45%iger Natronlauge [entspricht 3 Mol NaOH] versetzt. Man erhitzt in einem Autoklaven unter Eigendruck für 6 Stunden auf 190 bis 195°C. Das Reaktionsgemisch wird anschließend bei Raumtemperatur mit 2500 ml Wasser verdünnt. Man extrahiert dreimal mit je 300 ml MTBE, stellt die wässrige Phase mit konz. Salzsäure auf pH 1 und saugt den Feststoff ab. Nach Waschen mit zweimal je 1000 ml Wasser und Trocknen erhält man 139,7 g Feststoff mit einer Reinheit nach GC von 99%. Daraus resultiert eine Ausbeute von 84,2% der Theorie über 3 Schritte, d.h. im Durchschnitt ca. 94,5% der Theorie je Schritt.

## Patentansprüche

1. Verfahren zur Herstellung von 2,5-Dimethylphenylessigsäure, **dadurch gekennzeichnet, dass** man p-Xylol mit Chloracetylchlorid in einer Friedel-Crafts-Reaktion zu 2-Chlor-1-(2,5-dimethyl-phenyl)-ethanon umsetzt, welches mit der Verbindung der Formel (II)
HO-CH₂-X-CH₂-OH (II)
in welcher
X für eine direkte Einfachbindung, CH₂, CHCH₃, C(C₂H₅)₂, C(CH₃)₂ oder C(C₂H₅)₂ steht
zu der Verbindung der Formel (III) 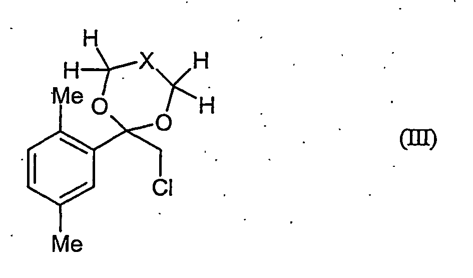 in welcher
X die oben angegebene Bedeutung hat
umgesetzt wird;
die Verbindung der Formel (III) wird anschließend zu einem Gemisch der Formeln (IV) und (V) 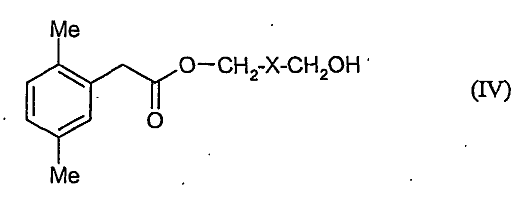 und 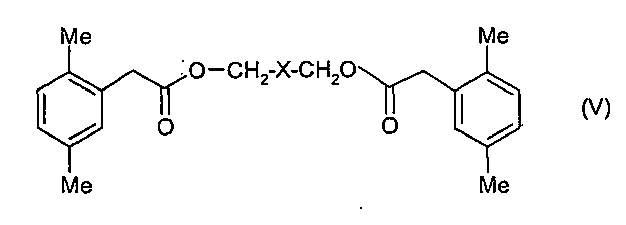 in welcher
X die oben angegebenen Bedeutungen hat,
umgelagert und diese dann zur 2,5-Dimethylphenylessigsäure verseift.

2. Verfahren zur Herstellung von 2,5-Dimethylphenylessigsäure gemäß Anspruch 1, wobei X für eine direkte Einfachbindung, CH₂, C(CH₃)₂ oder C(C₂H₅)₂ steht.

3. Verfahren zur Herstellung von 2,5-Dimethylphenylessigsäure gemäß Anspruch 1, wobei X für eine direkte Einfachbindung, C(CH₃)₂ oder C(C₂H₅)₂ steht.

4. Verbindung der Formel (III) 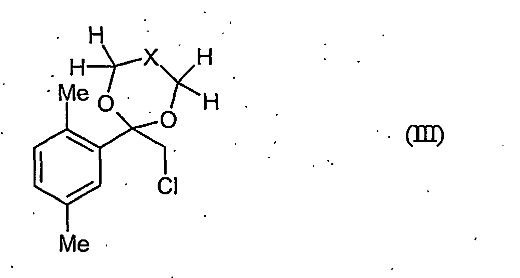 in welcher
X für eine direkte Einfachbindung, CH₂, CHCH₃, C(C₂H₅)₂, C(CH₃)₂ oder C(C₂H₅)₂ steht.

## Claims

1. Method for preparing 2,5-dimethylphenylacetic acid, **characterized in that** p-xylene is converted with chloroacetyl chloride in a Friedel-Crafts reaction into 2-chloro-1-(2,5-dimethylphenyl)ethanone, which is reacted with the compound of the formula (II)
HO-CH₂-X-CH₂-OH (II)
in which
X is a direct single bond, CH₂, CHCH₃, C(C₂H₅)₂, C(CH₃)₂ or C(C₂H₅)₂,
to give the compound of the formula (III) 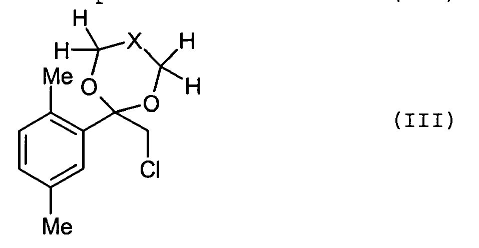 in which
X has the meaning indicated above;
the compound of the formula (III) is then rearranged to give a mixture of the formulae (IV) and (V) 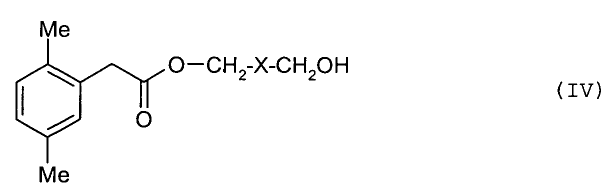 and 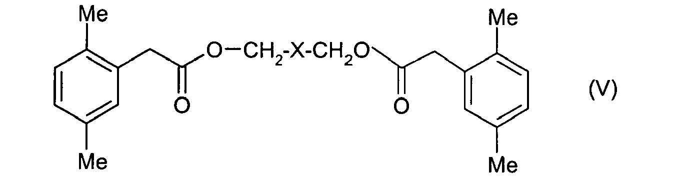 in which
X has the meanings indicated above,
and the latter is then hydrolyzed to 2,5-dimethylphenylacetic acid.

2. Method for preparing 2,5-dimethylphenylacetic acid according to Claim 1, X being a direct single bond, CH₂, C(CH₃) or C(C₂H₅)₂.

3. Method for preparing 2,5-dimethylphenylacetic acid according to Claim 1, X being a direct single bond, C(CH₃) or C(C₂H₅)₂.

4. Compound of the formula (III) 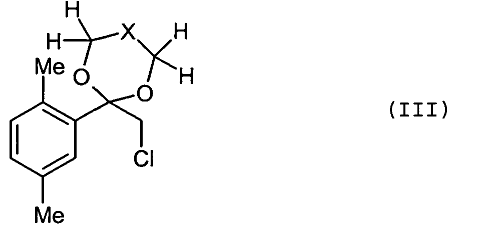 in which
X is a direct single bond, CH₂, CHCH₃, C(C₂H₅)₂, C(CH₃)₂ or C(C₂H₅)₂.

## Revendications

1. Procédé de production d'acide 2,5-diméthylphénylacétique, **caractérisé en ce qu'**on fait réagir du p-xylène avec du chlorure de chloracétyle dans une réaction de Friedel-Crafts pour former de la 2-chloro-1-(2,5-diméthyl-phényl)-éthanone qu'on fait réagir avec le composé de formule (II)
HO-CH₂-X-CH₂-OH (II)
dans laquelle
X représente une liaison simple directe, un reste CH₂, CHCH₃, C(C₂H₅)₂, C(CH₃)₂ ou C(C₂H₅)₂,
pour obtenir un composé de formule (III) 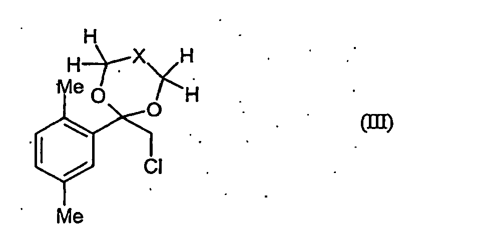 dans laquelle
X a la définition indiquée ci-dessus ;
on transpose ensuite le composé de formule (III) en un mélange de formules (IV) et (V) 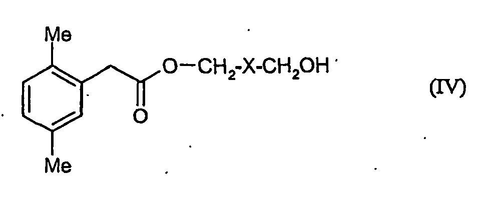 et 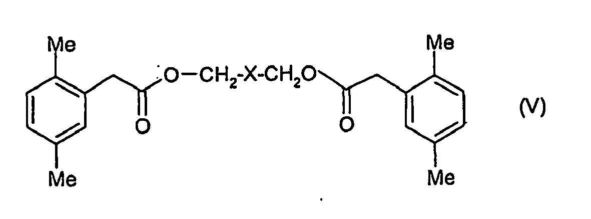 formules dans lesquelles
X a les définitions indiquées ci-dessus,
qu'on saponifie ensuite en acide 2,5-diméthylphénylacétique.

2. Procédé de production d'acide 2,5-diméthylphénylacétique suivant la revendication 1, dans lequel X représente une liaison simple directe, CH₂, C(CH₃)₂ ou C(C₂H₅)₂.

3. Procédé de production d'acide 2,5-diméthylphénylacétique suivant la revendication 1, dans lequel X représente une liaison simple directe, C(CH₃)₂ ou C(C₂H₅)₂.

4. Composé de formule (III) 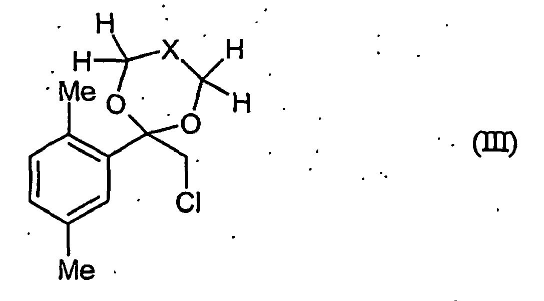 dans laquelle
X représente une liaison simple directe CH₂, CHCH₃, C(C₂H₅)₂, C(CH₃)₂ ou C(C₂H₅)₂.
